(19) Europäisches Patentamt
European Patent Office
Office européen des brevets

(11) Veröffentlichungsnummer: **0 001 255**
**B1**

(12) # EUROPÄISCHE PATENTSCHRIFT

(45) Veröffentlichungstag der Patentschrift: **30.09.81**

(51) Int. Cl.³: **C 07 C 177/00,**
A 61 K 31/557

(21) Anmeldenummer: **78100877.6**

(22) Anmeldetag: **13.09.78**

(54) **Neue fluorhaltige Prostaglandinderivate, Verfahren zu ihrer Herstellung und ihre Verwendung als Arzneimittel.**

(30) Priorität: **21.09.77 DE 2742407**

(43) Veröffentlichungstag der Anmeldung:
**04.04.79 Patentblatt 79/7**

(45) Bekanntmachung des Hinweises auf die Patenterteilung:
**30.09.81 Patentblatt 81/39**

(84) Benannte Vertragsstaaten:
**BE CH DE FR GB NL SE**

(56) Entgegenhaltungen:
**FR - A - 2 322 593**

(73) Patentinhaber: **HOECHST AKTIENGESELLSCHAFT**
**Postfach 80 03 20**
**D-6230 Frankfurt/Main 80 (DE)**

(72) Erfinder: **Bartmann, Wilhelm, Dr.**
**Am Dachsbau 5**
**D-6232 Bad Soden am Taunus (DE)**
Erfinder: **Beck, Gerard, Dr.**
**Gustav-Freytag-Strasse 34**
**D-6000 Frankfurt/Main (DE)**
Erfinder: **Lerch, Ulrich, Dr.**
**Schwalbenweg 19**
**D-6238 Hofheim am Taunus (DE)**
Erfinder: **Schölkens, Bernward, Dr.**
**Am Fliedergarten 1**
**D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 255 B1

Neue fluorhaltige Prostaglandinderivate, Verfahren zu ihrer Herstellung und ihre Verwendung als Arzneimittel

Prostaglandine sind eine Gruppe von Fettsäuren, die in zahlreichen Geweben und Organen von Mensch und Tier vorkommen. Das Grundgerüst der natürlichen Prostaglandine besteht aus 20 Kohlenstoffatomen, die in Form eines Fünfrings und zweier benachbarter linearer Seitenketten angeordnet sind.

Die pharmakologischen Effekte der Prostaglandine erstrekken sich u.a. auf die Gebiete der Reproduktion, des Bronchialmuskeltonus, des Blutdrucks und der Gastroenterologie. Die pharmakologischen Eigenschaften der natürlichen Prostaglandine sind Gegenstand zahlreicher Übersichtsartikel, z.B. N. H. Andersen und P. M. Ramwell in Arch. Internal Med. *133,* 30 (1974); R. L. Jones in Pathobiology Anm. 1972, 359; J. Pike in Scient. American *225,* 84 (1971) oder M. P. L. Caton in Progress in Med. Chem. vol. 8 ed.: Butterworth, London, 1971.

Die Synthesen von nicht natürlich vorkommenden Analogen von Prostansäuren, in denen die Vielzahl der pharmakologischen Wirkungen der natürlichen Prostaglandine differenziert sind, gewinnt zunehmend an Bedeutung.

Die vorliegende Erfindung betrifft neue, fluorhaltige Verbindungen der Formel I

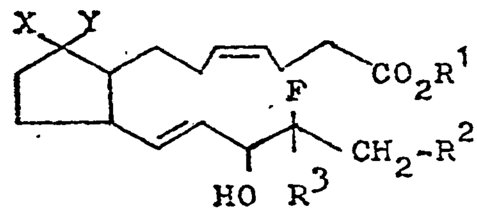

I

die strukturell mit den natürlichen Prostaglandinen verwandt sind und in welcher bedeuten

X und Y zusammen Sauerstoff oder jeweils Wasserstoff oder eine Hydroxylgruppe, wobei X und Y verschieden sind

$R^1$ einen geradkettigen oder verzweigten Alkylrest mit bis zu 8 Kohlenstoffatomen, in dem eine $CH_2$-Gruppe durch ein Sauerstoffatom oder ein Wasserstoffatom durch eine Hydroxylgruppe ersetzt ist, wobei die Sauerstoffunktion durch mindestens zwei Kohlenstoffatome von der Carboxylgruppe des Prostaglandins getrennt ist, oder einen Phenylalkylrest, wobei der Alkylrest bis zu 5 C-Atome enthält und eine $CH_2$-Gruppe durch ein Sauerstoffatom ersetzt ist

$R_2$ einen geraden oder verzweigten Alkylrest mit 3 bis 5 Kohlenstoffatomen, oder einen Cycloalkylrest mit 3 bis 7 Kohlenstoffatomen

$R_3$ Wasserstoff.

Unter den Substituenten $R^1$ sind die im folgenden aufgeführten besonders bevorzugt:

2-(Pent-3-yloxy)-äthyl, 3-(Äthoxy)-äthyl, 2-(Propoxy)-äthyl, 2-(Butoxy)-äthyl, 2-(Pentyl-oxy)-äthyl, 3-(Methoxy)-propyl, 3-(Äthoxy)-propyl, 3-(Propoxy)-propyl, 3-(Butoxy)-propyl, 3-(Butoxy)-propyl, 3-(Methyl-propoxy)-propyl, 3-(2-Butoxy)-propyl, 4-(Methoxy)-butyl, 4-(Äthoxy)-butyl, 4-(Propoxy)-butyl, 5-(Methoxy)-pentyl, 5-(Äthoxy)-pentyl, 6-(Methoxy)-hexyl, Benzyl, 2-Phenyläthyl, 3-Phenylpropyl, 4-Phenylbutyl, 5-Phenylpentyl, 2-Phenoxyäthyl, 3-Phenoxypropyl, 4-Phenoxybutyl, 5-Phenoxypentyl, 3-(Benzyloxy)-propyl, 2-Hydroxyäthyl, 3-Hydroxypropyl, 4-Hydroxybutyl, 3-Hydroxybutyl, 5-Hydroxypentyl, 4-Hydroxypentyl, 6-Hydroxyhexyl, 5-Hydroxyhexyl, 4-Hydroxyhexyl, 3-Hydroxyhexyl, 7-Hydroxyheptyl, 8-Hydroxyoctyl.

Unter den Substituenten $R^2$ sind die im folgenden aufgeführten besonders bevorzugt:

n-Propyl, 2-Propyl, n-Butyl, 2-Butyl, t-Butyl, n-Pentyl, 3-Pentyl, 3-Methylbutyl, Neopentyl, Cyclopentyl, Cyclohexyl, Cycloheptyl.

Gegenstand der Erfindung ist ferner ein Verfahren zur Herstellung von Verbindungen der Formel I, das dadurch gekennzeichnet ist, daß man

a) einen Aldehyd der Formel III

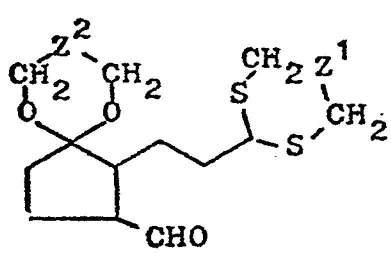

III

worin $Z^1$ und $Z^2$ eine $CH_2$-Gruppe, eine $-C(CH_3)_2-$ Gruppe oder eine Einfachbindung bedeuten, mit einem Phosphonat der Formel IV

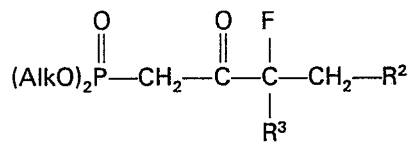

IV

worin Alk eine Alkylgruppe mit einem bis vier Kohlenstoffatomen bedeutet und $R^2$ und $R^3$ die zur Formel I angegebenen Bedeutungen haben, zu einem ungesättigten Keton der Formel V,

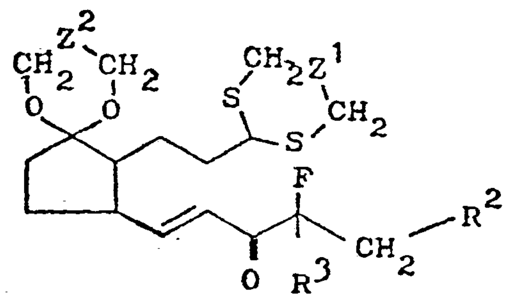

V

worin $R^2$ und $R^3$ die zur Formel I und $Z^1$ und $Z^2$ die zur Formel III angegebenen Bedeutungen haben, umsetzt.

b) das erhaltene Keton der Formel V zu einem Alkohol der Formel VI

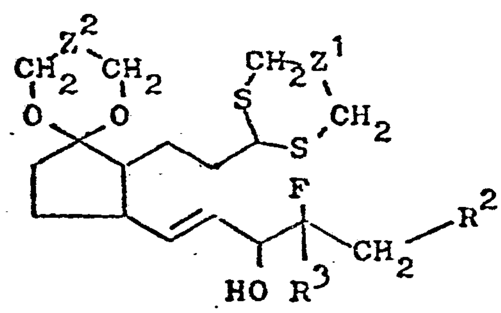

VI

worin $R^2$ und $R^3$ die zur Formel I und $Z^1$ und $Z^2$ die zur Formel III angegebenen Bedeutungen haben, reduziert,

c) die Alkoholfunktion der Verbindung VI mit einer unter sauren Bedingungen leicht abspaltbaren Gruppe schützt, wobei eine Verbindung der Formel VII

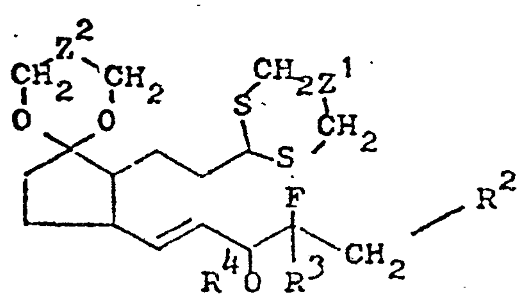

VII

worin $R^2$ und $R^3$ die zur Formel I, $Z^1$ und $Z^2$ die zur Formel III angegebenen Bedeutungen haben und $R^4$ eine leicht abspaltbare Schutzgruppe bedeutet, entsteht.

d) die Dithioacetalgruppe aus einer Verbindung der Formel VII schonend abspaltet, wobei ein Aldehyd der Formel VIII

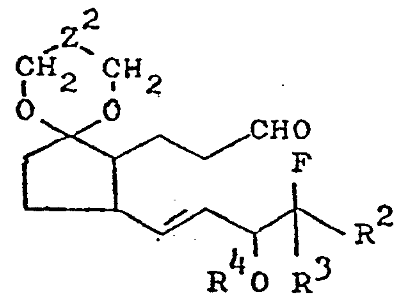

VIII

worin $R^2$ und $R^3$ die zur Formel I, $R^4$ die zur Formel VII und $Z^2$ die zur Formel III angegebenen Bedeutungen haben, ensteht

e) eine Verbindung der Formel VIII mit einem Ylid der Formel IX

$$(R^5)_3P{=}CH{-}(CH_2)_2{-}CO_2Me \qquad \text{IX}$$

worin die $R^5$ gleich oder verschieden sein können und geradkettiges ($C_1$—$C_4$)-Alkyl oder Phenyl und Me ein Alkalimetallatom bedeuten, zu einer Verbindung der Formel X

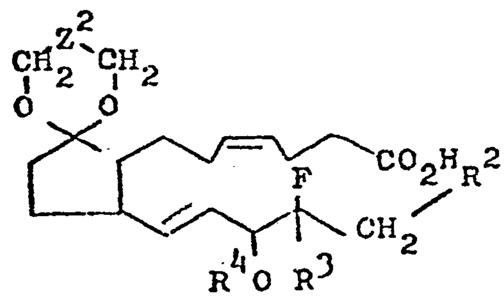

X

worin $R^2$ und $R^3$ die zur Formel I, $R^4$ die zur Formel VII und $Z^2$ die zur Formel III angegebenen Bedeutungen haben, umsetzt,

f) gegebenenfalls durch schonende saure Hydrolyse aus einer Verbindung der Formel X die Schutzgruppe $R^4$ entfernt, wobei eine Verbindung der Formel XI

XI

worin $R^2$ und $R^3$ die zur Formel I und $Z^2$ die zur Formel III angegebenen Bedeutungen haben, gebildet wird,

g) durch saure Hydrolyse einer Verbindung der Formel XI die Ketalschutzgruppe oder aus einer Verbindung der Formel X gleichzeitig die Schutzgruppe $R^4$ und die Ketalschutzgruppe entfernt, wobei Verbindungen der Formel I entstehen, worin X und Y zusammen Sauerstoff und $R^1$ Wasserstoff bedeuten und $R^2$ und $R^3$ die zur Formel I angegebenen Bedeutungen haben.

h) gegebenenfalls eine Verbindung der Formel I, worin X und Y zusammen Sauerstoff bedeutet und $R^1$, $R^2$ und $R^3$ die zur Formel I in Anspruch 1 angegebenen Bedeutungen haben, zu einer Verbindung der Formel I, worin X und Y verschieden sind und Wasserstoff oder eine Hydroxylgruppe bedeuten und $R^1$, $R^2$ und $R^3$ die zur Formel I in Anspruch 1 angegebenen Bedeutungen haben, reduziert,

i) gegebenenfalls eine Verbindung der Formel I, worin X, Y, $R^2$ und $R^3$ die zur Formel I in Anspruch 1 angegebenen Bedeutungen haben und $R^1$ Wasserstoff bedeutet, zu einer Verbindung der Formel I, worin X, Y, $R^1$, $R^2$ und $R^3$ die zur Formel I in Anspruch 1 angegebenen Bedeutungen haben, wobei $R^1$ jedoch nicht Wasserstoff bedeutet, verestert,

j) und gegebenenfalls eine Verbindung der Formel I, worin $R^2$, $R^3$, X und Y die zur Formel I in Anspruch 1 angegebenen Bedeutungen haben und $R^1$ Wasserstoff bedeutet, in ein physiologisch verträgliches Metalloder Aminsalz überführt.

Die Verfahrensschritte zur Herstellung von Verbindungen der Formel I ($R^1$=H) können durchgeführt werden in Analogie zu dem in der DE - A - 2 546 313 (HOE 75/F 270) beschriebenen Verfahren. Die Veresterung von Verbindungen der Formel I ($R^1$=H) erfolgt nach Methoden, wie sie in der DE - A - 2 628 564 (HOE 76/F 149) beschrieben sind.

Die erfindungsgemäßen Verbindungen der Formel I werden gewöhnlich in Form ihrer Racemate erhalten. Diese können gegebenenfalls nach den üblichen Methoden der Racemattrennung in Form der optisch aktiven Antipoden erhalten werden.

Die Ausgangsverbindungen der Formel III können gemäß der DE - A - 2 430 700 (HOE 74/F 178) hergestellt werden.

Außer den in den Beispielen genannten lassen sich insbesondere auch die folgenden Verbindungen herstellen:

16-Fluor-15-hydroxy-9-oxo-20-homo-(Z)-4, (E)-13-prostadiensäure-3-methoxypropylester
16-Fluor-15-hydroxy-9-oxo-20-(propyl-2)-(Z)-4, (E)-13-prostadiensäure-4-methoxybutylester
16-Fluor-15-hydroxy-9-oxo-(Z)-4, (E)-13-prostadiensäure-3-hydroxypropylester
17-Cyclopentyl-16-fluor-15-hydroxy-9-oxo-18,19,20-trinor-(Z)-4, (E)-13-prostadiensäure-2-isopropoxy-äthylester
9,15-Dihydroxy-16-fluor-16-methyl-(Z)-4, (E)-13-prostadiensäure-4-phenoxybutylester.

Aus der FR - A - 2 322 593 sind bereits ähnliche Prostaglandine der Formel

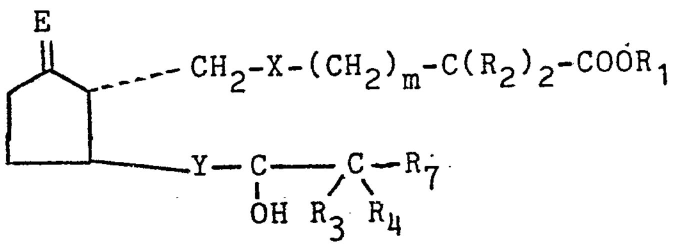

mit

E = Carbonyl, 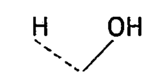 oder 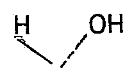

X = cis $-CH_2-CH=CH-$
Y = trans $-CH=CH-$
$R_1$ = H, Alkyl- oder Cycloalkylgruppe oder ein pharmakologisch verträgliches Kation
$R_2$ = H
$R_3$ = F; $R_4$ = H oder F und
$R_7$ = $-(CH_2)_n-CH_3$ mit n = 1 bis 5

mit prostaglandintypischen pharmakologischen Wirkungen bekannt.

Im Vergleich zu diesen Verbindungen zeichnen sich die erfindungsgemäßen Verbindungen jedoch aus durch eine wesentlich stärkere bronchospasmolytische Wirkung, wie durch Untersuchungen an männlichen Meerschweinchen mit der Versuchsanordnung nach Konzett-Rösler (Arch. exp. path. Pharmakol. 195 (1940), 71) festgestellt werden konnte.

Die erfindungsgemäßen Verbindungen zeichnen sich ferner durch blutdrucksenkende, magensaftsekretionshemmende, luteolytische und abortive, sowie andererseits durch spasmogene Eigenschaften aus. Verglichen mit den nicht-fluorierten Verbindungen sind sie stärker und länger anhaltend wirksam wie in Tierversuchen, z.B. an der Katze und am Meerschweinchen ermittelt wurde. Die Verbindungen können daher als Arzneimittel angewandt werden. Als Dosierungseinheit kommen 0,001 bis 1 mg, als Tagesdosis 0,005 bis 5 mg in Betracht.

Die erfindungsgemäßen Ester können in Form ihrer wäßrigen Lösungen oder Suspensionen oder auch gelöst oder suspendiert in pharmakologisch unbedenklichen organischen Lösungsmitteln wie ein- oder mehrwertige Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylenglykole oder auch in Gegenwart anderer pharmakologisch unbedenklicher Polymerträger wie z.B. Polyvinylpyrrolidon zur Anwendung gelangen.

Als Zubereitungen können die üblichen galenischen Infusionsder Injektionslösungen und Tabletten, sowie örtlich anwendbare Zubereitungen wie Cremes, Emulsionen, Suppositorien, insbesondere auch Aerosole in Frage kommen.

Eine weitere Anwendung der neuen Verbindungen liegt in der Kombination mit anderen Wirkstoffen. Neben anderen geeigneten Substanzen gehören dazu vor allem:

*Sekretolytica* wie z.B. Bisolvon, ß-*Sympathomimetica* wie z.B. Salbutamol oder Aludrin oder *Antitussiva* wie z.B. Codein, *fertilitätsregulierende Hormone* bzw. *Releasing-Hormone* wie LH, FSH, Östradiol, LH—RH, *Diuretika,* wie z.B. Furosemid, *Antidiabetika,* wie z.B. Glycodiazin, Tolbutamid, Glibenclamid, Phenformin, Buformin, Methformin, *Kreislaufmittel* im weitestesten Sinne, z.B. Coronardilatoren wie Chromonar oder Prenylamin, blutdrucksenkende Stoffe wie Reserpin, -Methyl-Dopa oder Clonidine oder Antiarrhythmika, *Lipidsenker,* Geriatrika und andere stoffwechselwirksame Präparate, *Psychopharmaka,* wie z.B. Chlordiazepoxid, Diazepam oder Meprobamat sowie *Vitamine,* oder Prostaglandine oder prostaglandinähnliche Verbindungen sowie auch Prostaglandinantagonisten und Prostaglandin-Biosynthesehemmer, wie z.B. nicht-steroidale Antiphlogistika.

Die Verbindungen der Formel V, VI, VII, VIII, IX, X und XI sind neue wertvolle Zwischenprodukte für die Herstellung von Verbindungen der Formel I.

Beispiel 1a

1-6-[(1,3-Dithia-2-cyclopentyl)-äthyl-]-1,4-dioxaspiro[4,4]non-7-yl-4-fluor-(E)-1-octen-3-on (V)

Zu 2,26 g einer 60% Suspension von Natriumhydrid in Mineralöl gibt man 120 ml absolutes Dimethyloxyäthan und läßt unter einer Stickstoffatmosphäre bei Raumtemperatur 16,8 g Dimethyl-3-fluor-2-oxoheptylphosphonat in 160 ml trockenem Dimethoxyäthan folgen. Man läßt bis zur Beendigung der Wasserstoffentwicklung rühren und gibt danach eine Lösung von 10,6 g 6-[(1,3-Dithia-2-cyclopentyl)-äthyl]-1,4-dioxaspiro[4,4]non-7-yl-carbaldehyd in 200 ml trockenem Dimethoxyäthan zu. Die Reaktionsmischung wird 20 Std. gerührt, mit Eisessig auf pH 6 gebracht und nach Zugabe von Aktivkohle durch ein Klärschichtfilter gesaugt. Das Filtrat wird i. Vak. eingedampft und der Rückstand an 250 g $SiO_2$ chromatographiert (1000 ml $CHCl_3$ und jeweils 500 ml $CHCl_3$/Äthylacetat-Gemische im Verhältnis 95:5 und 90:10).

NMR: 6,2—7,3 (m, 2) olefinische Protonen 3,9 (s, 4) $CH_2$—O; 3,2 (s, 4) $CH_2$—S

Beispiel 1b

1-6-[(1,3-Dithia-2-cyclopentyl)-äthyl]-1,4-dioxaspiro[4,4]non-7-yl-4-fluor-4-methyl-(E)-1-octen-3-on

Reaktion analog Beispiel 1a aus 6-[1,3-Dithia-2-cyclopentyl)-äthyl]-1,4-dioxaspiro[4,4]non-7-yl-carbaldehyd und Dimethyl-3-fluor-3-methyl-2-oxoheptylphosphonat.

NMR: 4,4 (t, 1) S—CH—S; 0,9 (t, 3) $CH_2$—$CH_3$

Beispiel 1c

1 - 6 - [(1,3 - Dithia - 2 - cyclopentyl) - äthyl] - 1,4 - dioxaspiro[4,4]non - 7 - yl - 7,7 - dimethyl - 4 - fluor - (E) - 1 - octen - 3 - on

Reaktion analog Beispiel 1a aus 6-[(1,3-Dithia-2-cyclopentyl)-äthyl]-1,4-dioxaspiro[4,4]non-7-yl-carbaldehyd und Dimethyl-6,6-dimethyl-3-fluor-2-oxoheptylphosphonat

NMR: 6,25—7,3 (m, 2) CH=CH 3,2 (s, 4) $CH_2$—S; 0,9 (s, 9) $CH_3$

Beispiel 1d

1 - 6 - [(1,3 - Dithia - 2 - cyclopentyl) - äthyl] - 1,4 - dioxaspiro[4,4]non - 7 - yl - 5 - cyclopentyl - 4 - fluor - (E) - 1 - penten - 3 - on

Reaktion analog Beispiel 1a aus 6-[(1,3-Dithia-2-cyclopentyl)-äthyl]-1,4-dioxaspiro[4,4]non-7-yl-carbaldehyd und Dimethyl-4-cyclopentyl-3-fluor-2-oxobutylphosphonat

NMR: 6,2—7,3 (m, 2) CH=CH; 3,95 (s, 4) $CH_2$—O 3,2 (s, 4) $CH_2$—S

Beispiel 1e

1-6-[(1,3-Dithia-2-cyclopentyl)-äthyl]-1,4-dioxaspiro[4,4]non-7-yl-4-äthyl-4-fluor-(E)-1-decen-3-on

Reaktion analog Beispiel 1a aus 6-[(1,3-Dithia-2-cyclopentyl)-äthyl]-1,4-dioxaspiro[4,4]non-7-yl-ylcarbaldehyd und Dimethyl-3-äthyl-3-fluor-2-oxononylphosphonat.

NMR: 3,15 (s, 4) $CH_2$—S; 3,95 (s, 4) $CH_2O$ 4,4 (t, 1) S—$CH_2$—S

Beispiel 1f

1-6-[(1,3-Dithia-2-cyclopentyl)-äthyl]-1,4-dioxaspiro[4,4]non-7-yl-4,4-difluor-(E)-1-octen-3-on

Reaktion analog Beispiel 1a aus 6-[(1,3-Dithia-2-cyclopentyl)-äthyl]-1,4-dioxaspiro[4,4]non-7-yl-carbaldehyd und Dimethyl-3,3-difluor-2-oxoheptylphosphonat.

NMR: 0,9 (t, 3) $CH_3$; 3,95 (s, 4) $CH_2$—O

Beispiel 2a

1-6-[(1,3-Dithia-2-cyclopentyl)-äthyl]-1,4-dioxaspiro[4,4]non-7-yl-4-fluor-(E)-1-octen-3-ol (VI)

0,2 Mol wasserfreies Zinkchlorid werden in 300 ml trockenem Dimethoxyäthan suspendiert und 0,8 Mol Natriumborhydrid portionsweise unter Argon zugegeben. Anschließend rührt man 1 Stunde bei Raumtemperatur. Man filtriert unter Feuchtigkeitsausschluß und tropft zu der erhaltenen Lösung 0,08 Mol 1-6-[(1,3-Dithia-2-cyclopentyl)-äthyl]-1,4-dioxaspiro[4,4]non-7-yl-4-fluor-(E)-1-octen-3-on in 50 ml trockenem Dimethoxyäthan. Man rührt 3 Std. bei Raumtemperatur und zersetzt dann das überschüssige Zinkchlorid mit Eisessig. Ggf. wird mit Triäthylamin neutralisiert, das Lösungsmittel i.Vak.

eingedampft und der Rückstand zwischen Chloroform und Wasser verteilt. Die organische Phase wird mit Magnesiumsulfat getrocknet, konzentriert und ggf. an Kieselgel chromatographiert.
NMR: 5,3—5,9 (m, 2) CH=CH; 0,95 (t, 3) $CH_3$

Beispiel 2b

1-6-[(1,3-Dithia-2-cyclopentyl)-äthyl]-1,4-dioxaspiro[4,4]non-7-yl-4-fluor-4-methyl-(E)-1-octen-3-ol

Reaktion analog Beispiel 2a aus 1-6-[(1,3-Dithia-2-cyclopentyl)-äthyl]-1,4-dioxaspiro[4,4]non-7-yl-4-fluor-4-methyl-(E)-1-octen-3-on.
NMR: 5,3—5,9 (m, 2) CH=CH; 4,4 (t, 1) S—CH—S 3,2 (s, 4) $CH_2$—S

Beispiel 2c

1 - 6 - [(1,3 - Dithia - 2 - cyclopentyl) - äthyl] - 1,4 - dioxaspiro[4,4]non - 7 - yl - 7,7 - dimethyl - 4 - fluor - (E) - 1 - octen - 3 - ol

Reaktion analog Beispiel 2 a aus 1-6-[(1,3-Dithia-2-cyclopentyl)-äthyl]-1,4-dioxaspiro[4,4]non-7-yl-7,7-dimethyl-4-fluor-(E)-1-octen-3-on.
NMR: 5,3—5,7 (m, 2) CH=CH; 3,95 (s, 4) $CH_2O$ 0,9 (s, 9) $CH_3$

Beispiel 2d

1 - 6 - [(1,3 - Dithia - 2 - cyclopentyl) - äthyl] - 1,4 - dioxaspiro[4,4]non - 7 - yl - 5 - cyclopentyl - 4 - fluor - (E) - 1 - penten - 3 - ol

Reaktion analog Beispiel 2a aus 1-6-[(1,3-Dithia-2-cyclopentyl)-äthyl]-1,4-dioxaspiro[4,4]non-7-yl-5-cyclopentyl-4-fluor-(E)-1-penten-3-on.
NMR: 5,3—5,8 (m, 2) CH=CH; 3,95 (s, 4) $CH_2O$ 3,2 (s, 4) $CH_2S$

Beispiel 2e

1-6-[(1,3-Dithia-2-cyclopentyl)-äthyl]-1,4-dioxaspiro[4,4]non-7-yl-4-äthyl-4-fluor-(E)-1-decen-3-ol

Reaktion analog Beispiel 2a aus 1-6-[(1,3-Dithia-2-cyclopentyl)-äthyl]-1,4-dioxaspiro[4,4]non-7-yl-4-äthyl-4-fluor-(E)-1-decen-3-on.
NMR: 5,3—5,9 (m, 2) CH=CH; 4,4 (t, 1) S—CH—S 3,95 (s, 4) $CH_2O$

Beispiel 2f

1-6-[(1,3-Dithia-2-cyclopentyl)-äthyl]-1,4-dioxaspiro[4,4]non-7-yl-4,4-difluor-(E)-1-octen-3-ol

Reaktion analog Beispiel 2a aus 1-6-[(1,3-Dithia-2-cyclopentyl)-äthyl]-1,4-dioxaspiro[4,4]non-7-yl-4,4-difluor-[E]-1-octen-3-on.
NMR: 5,3—5,9 (m, 2) CH=CH; 3,9 (s, 4) $CH_2O$

Beispiel 3a

1 - 6 - [(1,3 - Dithia - 2 - cyclopentyl) - äthyl] - 1,4 - dioxaspiro[4,4]non - 7 - yl - 4 - fluor - 3 - tetra - hydropyranyloxy - (E) - 1 - octen (VII)

Zu einer Lösung von 4,05 g (10 mMol) 1-6-[(1,3-Dithia-2-cyclopentyl)-äthyl]-1,4-dioxaspiro-[4,4]non-7-yl-4-fluor-(E)-1-octen-3-ol in 20 ml abs. Äther gibt man unter Argon und Feuchtigkeitsausschluß bei 0° 2 ml 2,3-Dihydropyran und 250 mg p-Toluol-sulfonsäure. Nach 10 Min. läßt man auf Raumtemperatur kommen und rührt noch 4 Stunden weiter. Es wird 0,3 ml Triäthylamin und 30 ml Äther zugegeben, mit Wasser gewaschen, getrocknet und i. Vak. eingedampft. Der Rückstand kann direkt weiter umgesetzt oder chromatographisch an 200 g $SiO_2$ gereinigt werden. Elutionsmittel: Chloroform (250 ml), Chloroform/Äthylacetat 95:5 (250 ml), 9:1 (500 ml) und schließlich 8:2. Ausbeute 86% Reinprodukt.
NMR: 4,7 (m, 1) O—CH—O; 5,3—5,9 (m, 2) CH=CH 3,95 (s, 4) $CH_2O$; 3,2 (s, 4) $CH_2S$

Beispiel 3b

1 - 6 - [(1,3 - Dithia - 2 - cyclopentyl) - äthyl] - 1,4 - dioxaspiro[4,4]non - 7 - yl - 4 - fluor - 4 - methyl - 3 - tetrahydropyranyloxy - (E) - 1 - octen

Reaktion analog Beispiel 3a aus 1-6-[(1,3-Dithia-2-cyclopentyl)-äthyl]-1,4-dioxaspiro[4,4]non-7-yl-4-fluor-4-methyl-(E)-1-octen-3-ol.
NMR: 4,7 (m, 1) O—CH—O; 4,4 (t, 1) S—CH—S 3,95 (s, 4) $CH_2O$

Beispiel 3c

1 - 6 - [(1,3 - Dithia - 2 - cyclopentyl) - äthyl] - 1,4 - dioxaspiro[4,4]non - 7 - yl - 7,7 - dimethyl - 4 - fluor - 3 - tetrahydropyranyloxy - (E) - 1 - octen

Reaktion analog Beispiel 3a aus 1-6-[(1,3-Dithia-2-cyclopentyl)-äthyl]-1,4-dioxaspiro[4,4]non-7-yl-7,7-dimethyl-4-fluor-(E)-1-octen-3-ol.
NMR: 4,65 (m, 1) O—CH—O; 5,3—5,9 (m, 2) CH=CH; 0,9 (s, 9) $CH_3$

Beispiel 3d

1 - 6 - [(1,3 - Dithia - 2 - cyclopentyl) - äthyl] - 1,4 - dioxaspiro[4,4]non - 7 - yl - 5 - cyclopentyl - 4 -

fluor - 3 - tetrahydropyranyloxy - (E) - 1 - penten

Reaktion analog Beispiel 3a aus 1-6-[(1,3-Dithia-2-cyclopentyl)-äthyl]-1,4-dioxaspiro[4,4]-non-7-yl-5-cyclopentyl-4-fluor-(E)-1-penten-3-ol.

NMR: 4,7 (m, 1) O—CHO; 3,95 (s, 4) $CH_2O$

Beispiel 3e

1 - 6 - [(1,3 - Dithia - 2 - cyclopentyl) - äthyl] - 1,4 - dioxaspiro[4,4]non - 7 - yl - 4 - äthyl - 4 - fluor - 3 - tetrahydropyranyloxy - (E) - 1 - decen

Reaktion analog Beispiel 3a aus 1-6-[(1,3-Dithia-2-cyclopentyl)-äthyl]-1,4-dioxaspiro[4,4]non-7-yl-4-äthyl-4-fluor-(E)-1-decen-3-ol.

Beispiel 3f

1 - 6 - [(1,3 - Dithia - 2 - cyclopentyl) - äthyl] - 1,4 - dioxaspiro[4,4]non - 7 - yl - 4,4 - difluor - 3 - tetrahydropyranyloxy - (E) - 1 - octen

Reaktion analog Beispiel 3a aus 1-6-[(1,3-Dithia-2-cyclopentyl)-äthyl]-1,4-dioxaspiro[4,4]non-7-yl-4,4-difluor-(E)-1-octen-3-ol.

NMR: 5,3—5,8 (m, 2) CH=CH; 4,7 (m, 1) O—CH—O 4,4 (t, 1) S—CH—S

Beispiel 4a

3 - [7 - (4 - Fluor - 3 - tetrahydropyranyloxy - (E) - 1 - octen - 1 - yl) - 1,4 - dioxaspiro[4,4]non - 6 - yl]-propionaldehyd (VIII)

Man rührt unter einer Argonatmosphäre 4,2 g 1-6-[(1,3-Dithia-2-cyclopentyl)-äthyl]-1,4-dioxaspiro[4,4]non-7-yl-4-fluor-3-tetrahydropyranyloxy-(E)-1-octen, 8,5 g Methyljodid und 6,8 g Calciumcarbonat in 40 ml DMF und 1,2 ml Wasser bei 50°. Nach 4—5 Stunden wird das Lösungsmittel i.Vak. entfernt und der Rückstand zwischen Wasser und Äther verteilt. Die organische Phase wird vom Ungelösten befreit, mit 2%iger Natriumthiosulfatlösung und Wasser gewaschen und schließlich das Lösungsmittel i.Vak. entfernt. Der Rückstand kann ohne weitere Reinigung für die folgende Reaktion eingesetzt werden oder, in Cyclohexan/Äthylacetat 7:3 gelöst durch etwa 60—70 g $SiO_2$ filtriert werden. Man erhält das Reinprodukt in 50—70%iger Ausbeute.

NMR: 9,8 (1) CH=O; 5,3—5,9 (m, 2) CH=CH 3,95 (s, 4) $CH_2O$

Beispiel 4b

3 - [7 - (4 - Fluor - 4 - methyl - 3 - tetrahydropyranyloxy - (E) - 1 - octen - 1 - yl) - 1,4 - dioxaspiro[4,4] - non - 6 - yl] - propionaldehyd

Reaktion analog Beispiel 4a aus 1-6-[(1,3-Dithia-2-cyclopentyl)-äthyl]-1,4-dioxaspiro[4,4]non-7-yl-4-fluor-4-methyl-3-tetrahydropyranyloxy-(E)-1-octen.

NMR: 9,7 (1) CH=O; 3,95 (s, 4) $CH_2O$ 5,3—5,8 (m, 2) CH=CH

Beispiel 4c

3 - [7 - (7,7 - Dimethyl - 4 - fluor - 3 - tetrahydropyranyloxy - (E) - 1 - octen - 1 - yl) - 1,4 - dioxaspiro - [4,4]non - 6 - yl] - propionaldehyd

Reaktion analog Beispiel 4a aus 1-6-[(1,3-Dithia-2-cyclopentyl)-äthyl]-1,4-dioxaspiro[4,4]non-7-yl-7,7-dimethyl-4-fluor-3-tetrahydropyranyloxy-(E)-1-octen.

NMR: 9,85 (1) CH=O; 5,3—5,8 (m, 2) CH=CH 0,9 (s, 9) $CH_3$

Beispiel 4d

3[7 - (5 - Cyclopentyl - 4 - fluor - 3 - tetrahydropyranyl - oxy - (E) - 1 - penten - 1 - yl) - 1,4 - dioxa - spiro[4,4] - non - 6 - yl] - propionaldehyd

Reaktion analog Beispiel 4a aus 1-6-[(1,3-Dithia-2-cyclopentyl)-äthyl]-1,4-dioxaspiro[4,4]non-7-yl-5-cyclopentyl-4-fluor-3-tetrahydropyranyloxy-(E)-1-penten.

NMR: 9,7 (1) CH=O; 4,65 (m, 1) O—CH—O 3,95 (s, 4) $CH_2O$

Beispiel 4e

3 - [7 - (4 - Äthyl - 4 - fluor - 3 - tetrahydropyranyloxy - (E) - 1 - decen - 1 - yl) - 1,4 - dioxaspiro[4,4] - non - 6 - yl] - propionaldehyd

Reaktion analog Beispiel 4a aus 1-6-[(1,3-Dithia-2-cyclopentyl)-äthyl]-1,4-dioxaspiro[4,4]-non-7-yl-4-äthyl-4-fluor-3-tetrahydropyranyloxy-(E)-1-decen.

NMR: 9,85 (1) CH=O; 4,7 (m, 1) O—CH—O 5,3—5,8 (m, 2) CH=CH

Beispiel 4f

3 - [7 - 4,4 - Difluor - 3 - tetrahydropyranyloxy - (E) - 1 octen - 1 - yl) - 1,4 - dioxaspiro[4,4]non - 6 - yl] - propionaldehyd

Reaktion analog Beispiel 4 a aus 1-6-[(1,3-Dithia-2-cyclopentyl)-äthyl]-1,4-dioxaspiro[4,4]non-7-yl-4,4-difluor-3-tetrahydropyranyloxy-(E)-1-octen.

NMR: 0,97 (1) CHO; 4,7 (m, 1) O—CH—O 0,9 (3) $CH_3$

Beispiel 5a

7 - [7 - (4 - Fluor - 3 - tetrahydropyranyloxy - (E) - 1 - octen - 1 - yl) - 1,4 - dioxaspiro[4,4]non - 6 - yl] - (Z) - 4 - heptensäure (X)

Unter einer Argonatmosphäre läßt man bei 70° 4 g (0,1 Mol) einer 60%igen Suspension von Natriumhydrid in Mineralöl mit 40 ml abs. DMSO reagieren. Nach Beendigung der Wasserstoffentwicklung kühlt man mit Eiswasser und gibt innerhalb von 5 Min unter Rühren 21,5 g (0,05 Mol) trockenes, gepulvertes 3-Carboxypropyltriphenylphosphoniumbromid zu und läßt 15 Minuten bei Raumtemperatur weiterrühren. Zu der roten Lösung des Phosphoniumylids tropft man sodann eine Lösung von 10,3 g 3-[7-(4-Fluor-3-tetrahydropyranyloxy-(E)-1-octen-1-yl)-1,4-dioxaspiro[4,4]non-6-yl]-propionaldehyd in 15 ml abs. DMSO. Nach einer Stunde wird mit 5%iger $NaHSO_4$-Lösung neutral gestellt, 100 ml gesättigte NaCl-Lösung zugefügt und mit 200 ml Äther überschichtet. Unter starkem Rühren und Kühlung auf 0 bis —5° säuert man mit 5%iger $NaHSO_4$-Lösung auf pH 3 an, trennt die wäßrige Phase sofort ab und extrahiert diese noch zweimal mit Äther. Die vereinigten Ätherauszüge werden mit wenig Wasser dreimal gewaschen, getrocknet und i.Vak. eingedampft. Beim Stehen in wenig Äther bei —20° können ggf. Nebenprodukte zur Kristallisation gebracht und abgetrennt werden. Das Rohprodukt kann direkt weiter umgesetzt oder chromatographisch gereinigt werden, z.B. an 200 g $SiO_2$ mit Cyclohexan/Äthylacetat/Eisessig 90:10:1 (500 ml) und 80:20:1 (1000 ml). Ausbeute 60—70%

NMR: 10,3 (s, 1) COOH, 5,2—5,6 (m, 2) und 5,6—5,9 (m, 2) olefinische Protonen 3,95 (s, 4) $CH_2O$

Beispiel 5b

7 - [7 - (4 - Fluor - 4 - methyl - 3 - tetrahydropyranyloxy - (E) - 1 - octen - 1 - yl) - 1,4 - dioxaspiro[4,4] - non - 6 - yl] - (Z) - 4 - heptensäure

Reaktion analog Beispiel 5a aus 3-[7-(4-Fluor-4-methyl-3-tetrahydropyranyloxy-(E)-1-octen-1-yl)-1,4-dioxaspiro[4,4]non-6-yl]-propionaldehyd.

NMR: 10,6 (s, 1) COOH, 5,2—5,6 (m, 2) und 5,6—5,9 (m, 2) olef. Protonen, 4,7 (m, 1) O—CH—O

Beispiel 5c

7 - [7 - (7,7 - Dimethyl - 4 - fluor - 3 - tetrahydropyranyloxy - (E) - 1 - octen - 1 - yl) - 1,4 - dioxaspiro - [4,4]non - 6 - yl] - (Z) - 4 - heptensäure

Reaktion analog Beispiel 5a aus 3-[7-(7,7-Dimethyl-4-fluor-3-tetrahydropyranyloxy-(E)-1-octen-1-yl)-1,4-dioxaspiro[4,4]non-6-yl]-propionylaldehyd.

NMR: 9,8 (s, 1) $CO_2H$, 5,2—5,9 (m, 4) olefin. Protonen

Beispiel 5d

7 - [7 - (5 - Cyclopentyl - 4 - fluor - 3 - tetrahydropyranyloxy - (E) - 1 - penten - 1 - yl) - 1,4 - dioxa - spiro[4,4]non - 6 - yl] - (Z) - 4 - heptensäure

Reaktion analog Beispiel 5a aus 3-[7-(5-Cyclopentyl-4-fluor-3-tetrahydropyranyloxy-(E)-1-penten-1-yl)-1,4-dioxaspiro[4,4]non-6-yl]-propionaldehyd.

NMR: 5,2—5,6 (m, 2) und 5,2—5,6 (m, 2) CH=CH

Beispiel 5e

7 - [7 - (4 - Äthyl - 4 - fluor - 3 - tetrahydropyranyloxy - (E) - 1 - decen - 1 - yl) - 1,4 - dioxaspiro[4,4] - non - 6 - yl] - (Z) - 4 - heptensäure

Reaktion analog Beispiel 5a aus 3-[7-(4-Äthyl-4-fluor-3-tetrahydropyranyloxy-(E)-1-decen-1-yl)-1,4-dioxaspiro[4,4]non-6-yl]-propionaldehyd.

NMR: 10,4 (s, 1) $CO_2H$; 5,2—5,9 (m, 4) CH=CH

Beispiel 5f

7 - [7 - (4,4 - Difluor - 3 - tetrahydropyranyloxy - (E) - 1 - octen - 1 - yl) - 1,4 - dioxaspiro[4,4]non - 6 - yl] - (Z) - 4 - heptensäure

Reaktion analog Beispiel 5a aus 3-[7-(4,4-Difluor-3-tetrahydropyranyloxy-(E)-1-octen-1-yl)-1,4-dioxaspiro[4,4]non-6-yl]-propionaldehyd.

NMR: 5,2—5,6 (m, 2) und 5,7—6,0 (m, 2) olef. Protonen

Beispiel 6a

16-Fluor-15-hydroxy-9-oxo-(Z)-4,(E)-13-prostadiensäure (I)

Man löst 3,7 g 7-[7-(4-Fluor-3-tetrahydro-pyranyloxy-(E)-1-octen-1-yl)-1,4-dioxaspiro[4,4]non-6-yl]-(Z)-4-heptensäure in 150 ml Äthanol und 38 ml 10%iger wäßriger Oxalsäurelösung, rührt 1 1/2 Std. bei 50° und läßt dann bei Raumtemperatur über Nacht stehen. Das Lösungsmittel wird i.Vak. verdampft und der Rückstand zwischen Wasser und Äther verteilt. Die wäßrige Phase wird noch zweimal mit Äther extrahiert, die vereinigten Ätherphasen mit gesättigter Kochsalzlösung gewaschen und eingedampft. Der Rückstand wird an 130 g Silicagel chromatographiert. Als Elutionsmittel dient Cyclohexan/Äthylacetat/Eisessig 90:10:1 (500 ml), 80:20:1 (500 ml), danach 70:30:1. Man erhält zwei

Isomere mit $R_F$-Werten (Cyclohexan-Äthylacetat/Eisessig 50:50:1) von ,030 ($\beta$-Isomeres und 0,25 (-Isomeres)
NMR: ( ) 7,8 (s, 2) OH; 5,2—5,55 (m, 2) 5,55—5,9 (m, 2) CH=CH
(ß): 7,6 (s, 2) OH; 5,25—5,55 (m, 2) und 5,55—5,8 (m, 2) CH=CH

Beispiel 6b

16-Fluor-15-hydroxy-16-methyl-9-oxo-(Z)-4,(E)-13-prostadiensäure

Reaktion analog Beispiel 6a aus 7-[7-(4-Fluor-4-methyl-3-tetrahydropyranyloxy-(E)-1-octen-1-yl)-1,4-dioxaspiro[4,4]non-6-yl]-(Z)]-4-heptensäure.
NMR: 7,3 (s, 2) OH; 5,2—5,6 (m, 2); 5,6—5,9 (m, 2) olef. Protonen

Beispiel 6c

19,19-Dimethyl-16-fluor-15-hydroxy-9-oxo-(Z)-4,(E)-13-prostadiensäure

Reaktion analog Beispiel 6a aus 7-[7-(7,7-Dimethyl-4-fluor-3-tetrahydropyranyloxy-(E)-1-octen-1-yl)-1,4-dioxaspiro[4,4]non-6-yl]-(Z)-4-heptensäure.
NMR: 7,7 (s, 2) OH; 5,2—5,9 (m, 4) CH=CH 0,9 (s, 9) $CH_3$

Beispiel 6d

17 - Cyclopentyl - 16 - fluor - 15 - hydroxy - 9 - oxo - 18,19,20 - trinor - (Z) - 4,(E) - 13 - prostadien - säure

Reaktion analog Beispiel 6a aus 7-[7-(5-Cyclopentyl-4-fluor-3-tetrahydropyranyloxy-(E)-1-penten-1-yl)-1,4-dioxaspiro[4,4]-non-6-yl]-(Z)-4-heptensäure.
NMR: 5,2—5,6 (m, 2) 5,6—5,9 (m, 2) CH=CH

Beispiel 6e

16 - Äthyl - 16 - fluor - 15 - hydroxy - 9 - oxo - 20 - dihomo - (Z) - 4,(E) - 13 - prostadiensäure

Reaktion analog Beispiel 6a aus 7-[7-(4-Äthyl-4-fluor-3-tetrahydropyranyloxy-(E)-1-decen-1-yl)-1,4-dioxaspiro[4,4]non-6-yl]-(Z)-4-heptensäure.
NMR: 7,1 (s, 2) OH; 5,2—5,9 (m, 4) CH=CH

Beispiel 7a

9,15-Dihydroxy-16-fluor-(Z)-4,(E)-13-prostadiensäure

1,55 g 16-Fluor-15-hydroxy-9-oxo-(Z)-4,(E)-13-prostadiensäure (unpolares $\beta$-Isomeres) wird in 50 ml abs. Äthanol gelöst und unter Eiskühlung portionsweise 1,93 g Natriumborhydrid zugegeben. Nach 5 Stunden wird das Lösungsmittel i.Vak. entfernt, der Rückstand mit 100 ml gesättigter Kochsalzlösung versetzt und mit $NaH_2PO_4$-Lösung auf pH 3—4 angesäuert. Das Produkt wird mit Äther extrahiert, die Ätherphase mit Kochsalzlösung gewaschen, getrocknet und eingedampft.

Das Produkt wird durch Chromatographie 60 g $SiO_2$ in die beiden Isomeren aufgetrennt (Laufmittel: Cyclohexan/Äthylacetat/Eisessig 80:20:1 (500 ml), danach 70:30:1.
NMR: 5,2—5,8 (m, 4) CH=CH, überlagert von 5,5 (s, 3) OH 3,95 (m, 2) *H*C—OH

Auf die gleiche Art und Weise wird das polare ( )-Isomere der 16-Fluor-15-hydroxy-9-oxo-(Z)-4-,(E)-13-prostadiensäure reduziert.
NMR: 5,0—5,8 (m, 7) CH=CH und OH, 0,9 (3) $CH_3$

Beispiel 7b

9,15-Dihydroxy-16-fluor-16-methyl-(Z)-4,(E)-13-prostadiensäure

Reaktion analog Beispiel 7a aus 16-Fluor-15-hydroxy-16-methyl-9-oxo-(Z)-4,(E)-13-prostadiensäure.
NMR: 5,2—5,8 (m, 4 und s, 3) CH=CH und *OH* 3,95 (m, 2) *H*C—OH

Beispiel 7c

17 - Cyclopentyl - 9,15 - dihydroxy - 16 - fluor - 18,19,20 - trinor - (Z) - 4,(E) - 13 - prostadien - säure

Reaktion analog Beispiel 7a aus 17-Cyclopentyl-16-fluor-15-hydroxy-9-oxo-18,19,20-trinor-(Z)-4,(E)-13-prostadiensäure
NMR: 5,2—5,8 (m, 4) CH=CH, überlagert von 5,3 (s, 3) OH

Beispiel 8a

16 - Fluor - 15 - hydroxy - 9 - oxo - (Z) - 4,(E) - 13 - prostadiensäure - 4 - äthoxybutylester

100 mg 16-Fluor-15-hydroxy-9-oxo-(Z)-4,(E)-13-prostadiensäure wird in 2 ml abs. DMF gelöst, mit 19 mg Natriummethylat und 90 mg 4-Äthoxybutyljodid versetzt und unter Argon 6 Stunden bei 50° gerührt. Ggf. wird, wenn die Reaktionslösung neutral reagiert und noch Ausgangsmaterial vorhanden ist (DC), nochmals Natriummethylat und 4-Äthoxybutyljodid zugegeben.

Die Reaktionslösung wird zwischen Toluol und Wasser verteilt, die org. Phase mit Wasser

gewaschen, getrocknet und eingedampft. Durch Chromatographie an 6 g Silicagel erhält man den reinen Ester (Elutionsmittel Tetrachlorkohlenstoff/Aceton 9:1).
NMR: 5,2—5,5 (m, 2) und 5,5—5,8 (m, 2) CH=CH 3,2—3,7 (m, 4) $CH_2$—O—$CH_2$

Beispiel 8b

16-Fluor-15-hydroxy-9-oxo-(Z)-4,(E)-13-prostadiensäure-6-hydroxyhexylester

Reaktion analog Beispiel 8a aus 16-Fluor-15-hydroxy-9-oxo-(Z)-4,(E)-13-prostadiensäure und 6-Jodhexanol.
NMR: 5,2—5,5 (m, 2) und 5,5—5,8 (m, 2) CH=CH 4,1 (m, 1 und t, 2) *CH*—OH und $CO_2CH_2$ 3,65 $CH_2$—OH

Beispiel 8c

16-Fluor-15-hydroxy-9-oxo-(Z)-4,(E)-13-prostadiensäure-2-äthoxyäthylester

Reaktion aus 16-Fluor-15-hydroxy-9-oxo-(Z)-4,(E)-13-prostadiensäure und 2-Äthoxydiazoäthan.
NMR: 3,9—4,25 (m, 3) $CO_2CH_2$ und *CH*—OH 3,4—3,7 (m, 4) $CH_2$—O—$CH_2$

Beispiel 8d

17 - Cyclopentyl - 16 - fluor - 15 - hydroxy - 9 - oxo - 18,19,20 - trinor - (Z) - 4,(E) - 13 - prostadien - säure - 3 - phenoxypropylester

Reaktion analog Beispiel 8a aus 17-Cyclopentyl-16-fluor-15-hydroxy-9-oxo-18,19,20-trinor-(Z)-4,(E)-13-prostadiensäure und 3-Phenoxypropyljodid.
NMR: 3,8—4,3 (m, 5) O—$CH_2$ und *CH*—OH

**Patentansprüche**

1. Verbindungen der Formel I

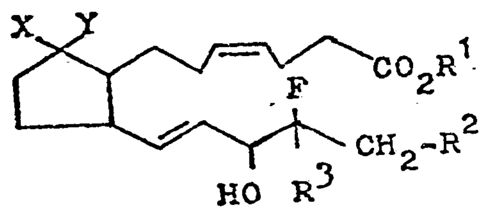

I

die strukturell mit den natürlichen Prostaglandinen verwandt sind und in welcher bedeuten

X und Y zusammen Sauerstoff oder jeweils Wasserstoff oder eine Hydroxylgruppe, wobei X und Y verschieden sind

$R^1$ einen geradkettigen oder verzweigten Alkylrest mit bis zu 8 Kohlenstoffatomen, in dem eine $CH_2$-Gruppe durch ein Sauerstoffatom oder ein Wasserstoffatom durch eine Hydroxylgruppe ersetzt ist, wobei die Sauerstoffunktion durch mindestens zwei Kohlenstoffatome von der Carboxylgruppe des Prostaglandins getrennt ist, oder einen Phenalkylrest, wobei der Alkylrest bis zu 5 C-Atome enthält und eine $CH_2$-Gruppe durch ein Sauerstoffatom ersetzt ist,

$R^2$ einen geraden oder verzweigten Alkylrest mit 3 bis 5 Kohlenstoffatomen, oder einen Cycloalkylrest mit 5 bis 7 Kohlenstoffatomen,

$R^3$ Wasserstoff.

2. Verfahren zur Herstellung von Verbindungen der Formel I in Anspruch 1, dadurch gekennzeichnet, daß man

a) einen Aldehyd der Formel III

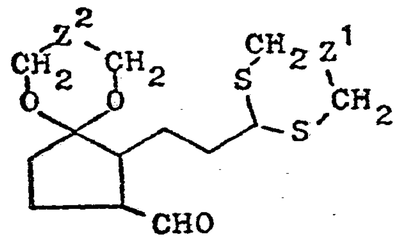

III

worin $Z^1$ und $Z^2$ eine $CH_2$-Gruppe, eine —$C(CH_3)_2$-Gruppe oder eine Einfachbindung bedeuten, mit einem Phosphonat der Formel IV

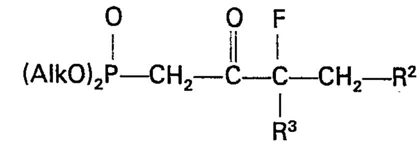

IV

worin Alk eine Alkylgruppe mit einem bis vier Kohlenstoffatomen bedeutet und $R^2$ und $R^3$ die zur Formel I angegebenen Bedeutungen haben, zu einem ungesättigten Keton der Formel V.

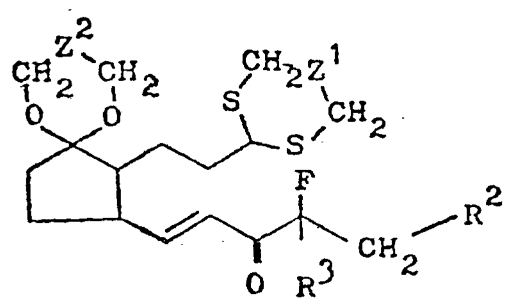

V

worin $R^2$ und $R^3$ die zur Formel I und $Z^1$ und $Z^2$ die zur Formel III angegebenen Bedeutungen haben, umsetzt.

b) das erhaltene Keton der Formel V zu einem Alkohol der Formel VI

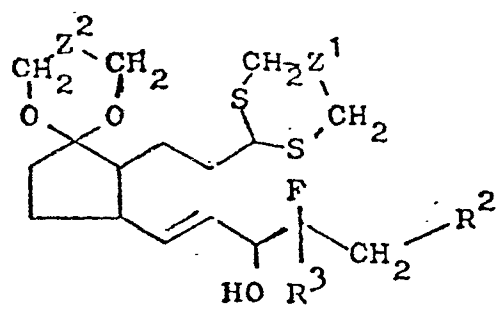

VI

worin $R^2$ und $R^3$ die zur Formel I und $Z^1$ und $Z^2$ die zur Formel III angegebenen Bedeutungen haben, reduziert,

c) die Alkoholfunktion der Verbindung VI mit einer unter sauren Bedingungen leicht abspaltbaren Gruppe schützt, wobei eine Verbindung der Formel VII

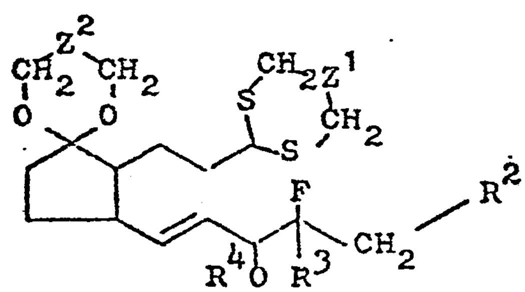

VII

worin $R^2$ und $R^3$ die zur Formel I, $Z^1$ und $Z^2$ die zur Formel III angegebenen Bedeutungen haben und $R^4$ eine leicht abspaltbare Schutzgruppe bedeutet, entsteht.

d) die Dithioacetalgruppe aus einer Verbindung der Formel VII schonend abspaltet, wobei ein Aldehyd der Formel VIII

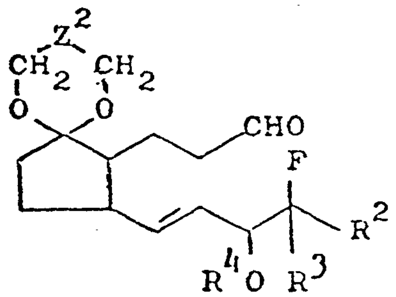

VIII

worin $R^2$ und $R^3$ die zur Formel I, $R^4$ die zur Formel VII und $Z^2$ die zur Formel III angegebenen Bedeutungen haben, ensteht

e) eine Verbindung der Formel VIII mit einem Ylid der Formel IX

$$(R^5)_3P{=}CH{-}(CH_2)_2{-}CO_2Me \qquad IX$$

worin die $R^5$ gleich oder verschieden sein können und geradkettiges $(C_1{-}C_4)$-Alkyl oder Phenyl und Me ein Alkalimetallatom bedeuten, zu einer Verbindung der Formel X

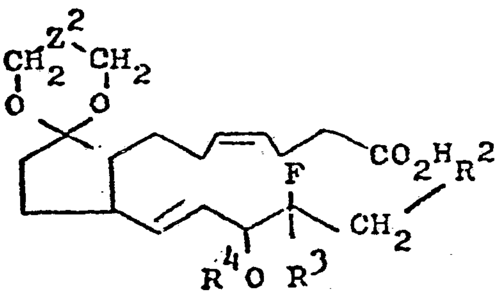

X

worin $R^2$ und $R^3$ die zur Formel I, $R^4$ die zur Formel VII und $Z^2$ die zur Formel III angegebenen Bedeutungen haben, umsetzt,

f) gegebenenfalls durch schonende saure Hydrolyse aus einer Verbindung der Formel X die Schutzgruppe $R^4$ entfernt, wobei eine Verbindung der Formel IX

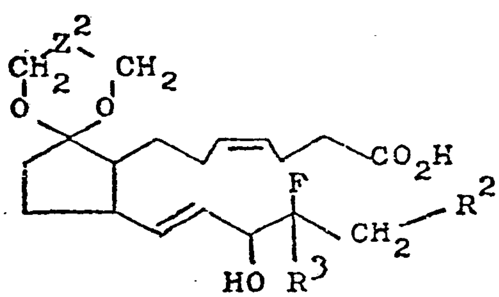

XI

worin $R^2$ und $R^3$ die zur Formel I und $Z^2$ die zur Formel III angegebenen Bedeutungen haben, gebildet wird,

g) durch saure Hydrolyse einer Verbindung der Formel XI die Ketalschutzgruppe oder aus einer Verbindung der Formel X gleichzeitig die Schutzgruppe $R^4$ und die Ketalschutzgruppe entfernt, wobei Verbindungen der Formel I entstehen, worin X und Y zusammen Sauerstoff und $R^1$ Wasserstoff bedeuten und $R^2$ und $R^3$ die zur Formel I angegebenen Bedeutungen haben.

h) gegebenenfalls eine Verbindung der Formel I, worin X und Y zusammen Sauerstoff bedeutet und $R^1$, $R^2$ und $R^3$ die zur Formel I in Anspruch 1 angegebenen Bedeutungen haben, zu einer Verbindung der Formel I, worin X und Y verschieden sind und Wasserstoff oder eine Hydroxylgruppe bedeuten und $R^1$, $R^2$ und $R^3$ die zur Formel I in Anspruch 1 angegebenen Bedeutungen haben, reduziert,

i) gegebenenfalls eine Verbindung der Formel I, worin X, Y, $R^2$ und $R^3$ die zur Formel I in Anspruch 1 angegeben Bedeutungen haben und $R^1$ Wasserstoff bedeutet, zu einer Verbindung der Formel I, worin X, Y, $R^1$, $R^2$ und $R^3$ die zur Formel I in Anspruch 1 angegebenen Bedeutungen haben, wobei $R^1$ jedoch nicht Wasserstoff bedeutet, verestert,

j) und gegebenenfalls eine Verbindung der Formel I, worin $R^2$, $R^3$, X und Y die zur Formel I in Anspruch 1 angegebenen Bedeutungen haben und $R^1$ Wasserstoff bedeutet, in ein physiologisch verträgliches Metalloder Aminsalz überführt.

3. Verfahren zur Herstellung von Arzneimitteln, dadurch gekennzeichnet, daß man eine Verbindung der in Anspruch 1 genannten Formel I, gegebenenfalls mit üblichen pharmazeutischen Trägern und/oder Stabilisatoren, in eine therapeutisch geeignete Darreichungsform bringt.

4. Arzneimittel, gekennzeichnet durch einen Gehalt an einer Verbindung der in Anspruch 1 genannten Formel I.

5. Verwendung einer Verbindung der in Anspruch 1 genannten Formel I als Arzneimittel.

**Revendications**

1. Composés de formule I

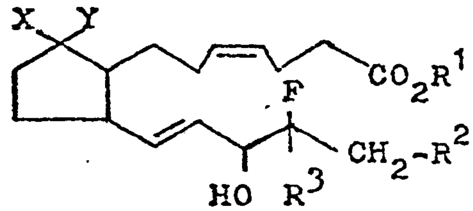

I

qui sont apparentés structurellement aux prostaglandines naturelles et dans lesquels:

X et Y représentent ensemble l'oxygène ou chacun l'hydrogène ou un groupe hydroxyle, X et Y étant alors différents,

$R^1$ représente un radical alkyle à chaîne droite ou ramifiée ayant jusqu'à 8 atomes de carbone, dans lequel un groupe $CH_2$ est remplacé par un atome d'oxygène ou un atome d'hydrogène par un groupe hydroxyle, la fonction oxygène étant séparée du groupe carboxyle de la prostaglandine par au moins deux atomes de carbone, ou un radical phénylalkyle, la partie alkyle ayant jusqu'à 5 atoms de carbone et un groupe $CH_2$ étant remplacé par un atome d'oxygène,

$R^2$ représente un radical alkyle linéaire ou ramifié ayant de 3 à 5 atomes de carbone, ou un radical cycloalkyle ayant de 5 à 7 atomes de carbone, et

$R^3$ représente l'hydrogène.

2. Procédé de préparation de composés de formule I selon la revendication 1, caractérisé en ce que:

a) on fait réagir un aldéhyde de formule III

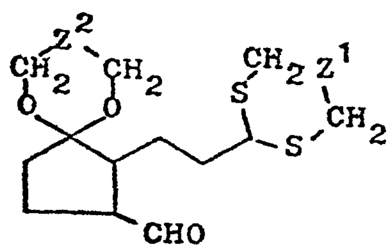

III

où $Z^1$, $Z^2$ représentent un groupe $CH_2$, un groupe $—C(CH_3)_2—$ ou une simple liaison, avec un phosphonate de formule IV

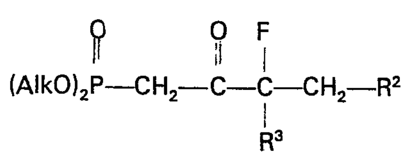

IV

où Alk représente un groupe alkyle ayant de 1 à 4 atomes de carbone et $R^2$ et $R^3$ ont les significations indiquées pour la formule I, afin d'obtenir une cétone insaturée de formule V

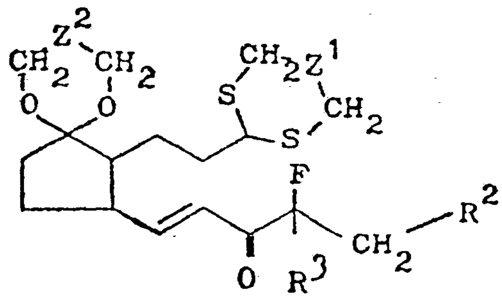

V

où $R^2$ et $R^3$ ont les significations indiquées pour la formule I et $Z^1$ et $Z^2$ les significations indiquées pour la formule III;

b) On réduit la cétone de formule V obtenue en un alcool de formule VI

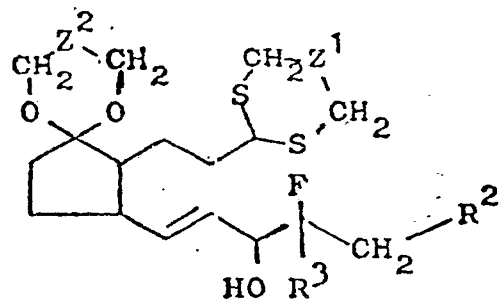

VI

où $R^2$ et $R^3$ ont les significations indiquées pour la formule I et $Z^1$ et $Z^2$ les significations indiquées pour la formule III;

c) On protège la fonction alcool du composé VI par un groupe facilement séparable dans des conditions acides, auquel cas il se forme un composé de formule VII

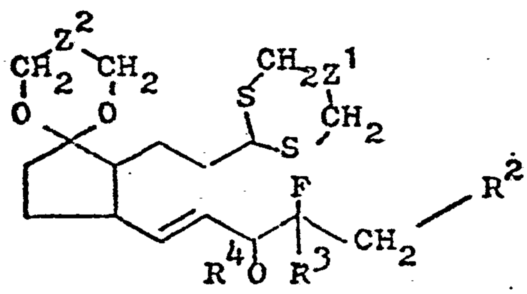

VII

où $R^2$ et $R^3$ ont les significations indiquées pour la formule I, $Z^1$ et $Z^2$ les significations indiquées pour la formule III et $R^4$ représente un groupe protecteur facilement séparable.

d) On sépare dans des conditions modérées, le groupe dithioacétal d'un composé de formule VII, auquel cas il se forme un aldéhyde de formule VIII

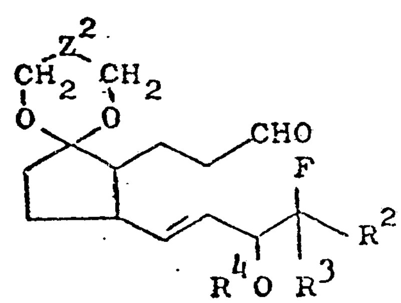

VIII

où $R^2$ et $R^3$ ont les significations indiquées pour la formule I, $R^4$ a la signification indiquée pour la formule VII et $Z^2$ la signification indiquée pour la formule III.

e) On fait réagir un composé de formule VIII avec un ylide de formule IX

$(R^5)_3P{=}CH{-}(CH_2)_2{-}CO_2Me$ IX

où les groupes $R^5$ peuvent être identiques ou différents et représentent un radical alkyle à chaîne droite en $C_1$—$C_4$ ou un radical phényle et Me représente un atome de métal alcalin, pour obtenir un composé de formule X

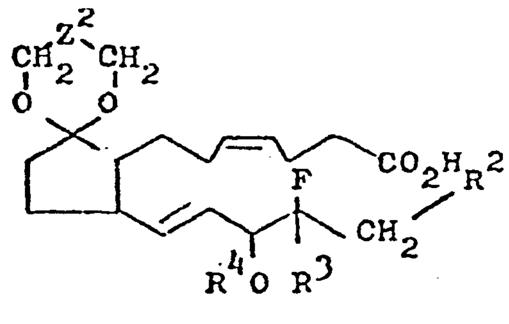

X

où $R^2$ et $R^3$ ont les significations indiquées pour la formule I, $R^4$ a la signification indiquée pour la formule VII et $Z^2$ la signification indiquée pour la formule III.

f) par une hydrolyse acide ménagée, on sépare éventuellement le groupe protecteur $R^4$ d'un composé de formule X, auquel cas il se forme un composé de formule XI

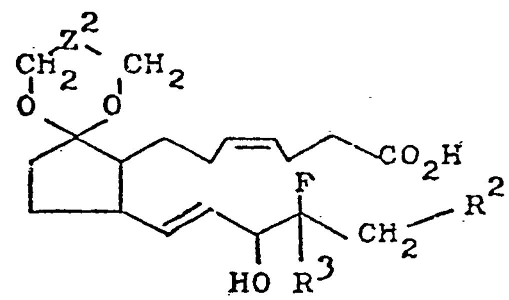

XI

où $R^2$ et $R^3$ ont les significations indiquées pour la formule I et $Z^2$ a la signification indiquée pour la formule III;

g) par une hydrolyse acide, on sépare d'un composé de formule XI, le groupe cétal-protecteur ou d'un composé de formule X simultanément le groupe protecteur $R^4$ et le groupe cétal-protecteur, auquel cas il se forme des composés de formule I dans lesquels X et Y représentent ensemble l'oxygène et $R^1$ l'hydrogène et $R^2$ et $R^3$ ont les significations indiquées pour la formule I;

h) on réduit éventuellement un composé de formule I dans lequel X et Y représentent ensemble l'oxygène et $R^1$, $R^2$ et $R^3$ ont les significations indiquées pour la formule I dans la revendication 1, en un composé de formule I dans lequel X et Y sont différents et représentent l'hydrogène ou un groupe hydroxyle et $R^1$, $R^2$ et $R^3$ ont les significations indiquées pour la formule I dans la revendication 1;

i) on estérifie éventuellement un composé de formule I dans lequel X, Y, $R^2$ et $R^3$ ont les significations indiquées pour la formule I dans la revendication 1 et $R^1$ représente l'hydrogène, en un composé de formule I où X, Y, $R^1$, $R^2$ et $R^3$ ont les significations indiquées pour la formule I dans la revendication 1, auquel cas $R^1$ ne représente cependant pas l'hydrogène;

j) et on transforme éventuellement un composé de formule I dans lequel $R^2$, $R^3$, X et Y ont les significations indiquées pour la formule I selon la revendication 1 et $R^1$ représente l'hydrogène, en un sel métallique ou un sel d'amine physiologiquement acceptable.

3. Procédé de préparation de médicaments, caractérisé en ce qu'on met sous une forme d'administration thérapeutiquement appropriée un composé de formule I selon la revendication 1, avec éventuellement des véhicules et ou des stabilisants pharmaceutiques usuels.

4. Médicament, caractérisé en ce qu'il contient un composé de formule I selon la revendication 1.

5. Utilisation d'un composé de formule I selon la revendication 1, comme médicament.

**Claims**

1. Compounds of the formula I

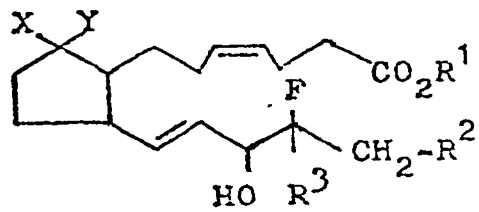

(I)

in which X and Y together denote oxygen or each denote hydrogen or a hydroxyl group, in which case X and Y are different, $R^1$ denotes a straight-chain, or branched alkyl radical with up to 8 carbon atoms, in which one $CH_2$ groups is replaced by an oxygen atom, or a hydrogen atom can be replaced by a hydroxyl group, the oxygen functional group being separated by at least two carbon atoms from the carboxyl group of the prostaglandin, or a phenalkyl radical in which the alkylene group has up to 5 carbon atoms and one of the $CH_2$ groups is replaced by an oxygen atom; $R^2$ denotes a straight-chain, branched, alkyl radical with three to five carbon atoms or a cycloalkyl radical with five to seven carbon aroms and $R^3$ denotes hydrogen.

2. A process for the manufacture of compounds of the formula I, which comprises

a) reacting an aldehyde of the formula III

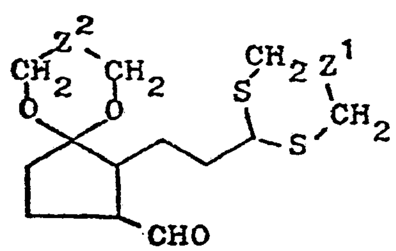

(III)

wherein

$Z^1$ and $Z^2$ denote a $CH_2$ group, a $—C(CH_3)_2$ group or a single bond, with a phosphonate of the formula IV

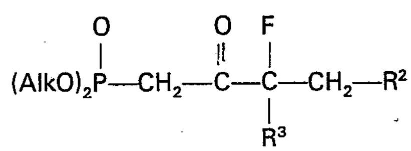

(IV)

wherein

Alk denotes an alkyl group with one to four carbon atoms and $R^2$ and $R^3$ are as defined for formula I, to give an unsaturated ketone of the formula V

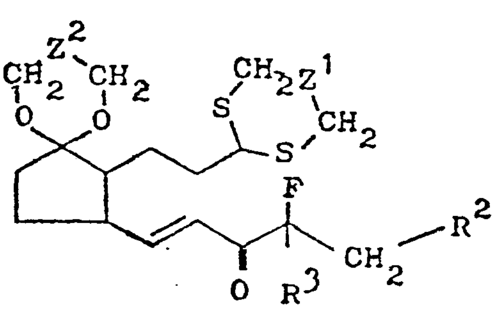

(V)

wherein

$R^2$ and $R^3$ are as defined for formula I and $Z^1$ and $Z^2$ are as defined for formula III,

b) reducing the resulting ketone of the formula V to an alcohol of the formula VI

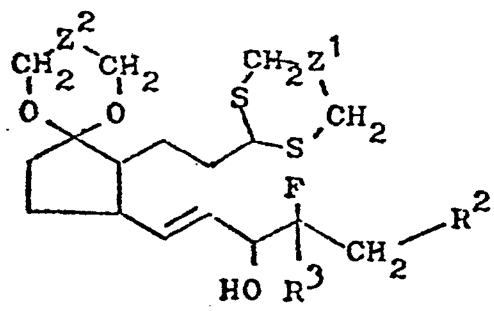

(VI)

wherein

$R^2$ and $R^3$ are as defined for formula I and $Z^1$ and $Z^2$ are as defined for formula III,

c) protecting the alcohol functional group in compound VI with a group which is easily detachable under acid conditions, a compound of the formula VII

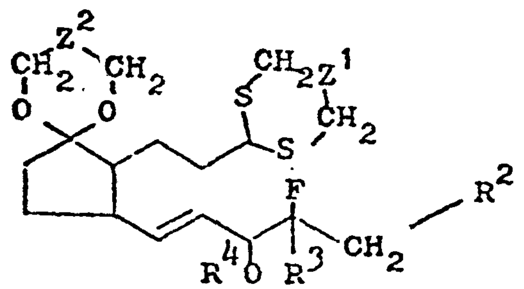

(VII)

wherein

$R^2$ and $R^3$ are as defined for formula I, $Z^1$ and $Z^2$ are as defined for formula III and $R^4$ denotes an easily detachable protective group, being formed,

d) detaching the dithioacetal group from a compound of the formula VII under gentle conditions, an aldehyde of the formula VIII

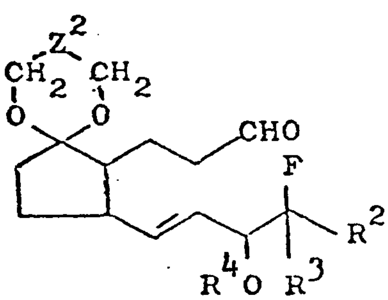

(VIII)

wherein

$R^2$ and $R^3$ are as defined for formula I, $R^4$ is as defined for formula VII and $Z^2$ is as defined for formula III, being formed,

e) reacting a compound of the formula VIII with an ylide of the formula IX

$$(R^5)_3P{=}CH{-}(CH_2)_2{-}CO_2Me \qquad (IX)$$

wherein

the $R^5$s can be identical or different and denote straight-chain $(C_1{-}C_4)$-alkyl or phenyl and Me denotes an alkali metal atom, to give a compound of the formula X

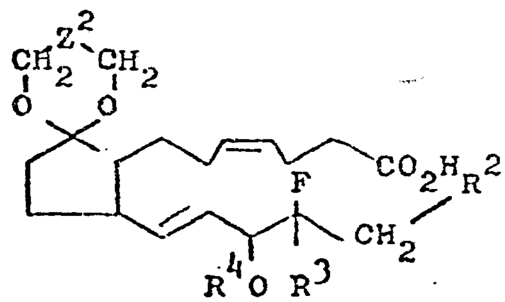

(X)

wherein

$R^2$ and $R^3$ are as defined for formula I, $R^4$ is as defined for formula VII and $Z^2$ is as defined for formula III,

f) optionally removing the protective group $R^4$ from a compound of the formula X by gentle acid hydrolysis, a compound of the formula XI

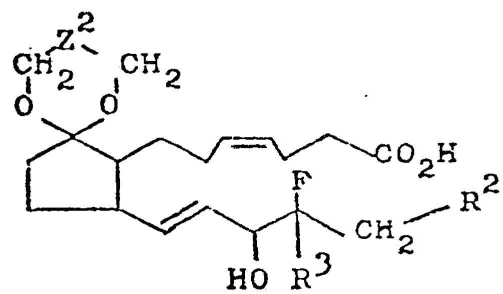

(XI)

wherein

$R^2$ and $R^3$ are as defined for formula I and $Z^2$ is as defined for formula III, being formed,

g) removing the ketal protective group by acid hydrolysis of a compound of the formula XI, or simultaneously removing the protective group $R^4$ and the ketal protective group from a compound of the formula X, compounds of the formula I wherein X and Y together denote oxygen and $R^1$ denotes hydrogen and $R^2$ and $R^3$ are as defined for formula I being formed,

h) optionally reducing a compound of the formula I wherein X and Y together denote oxygen and $R^1$, $R^2$ and $R^3$ are as defined for formula I in claim 1 to a compound of the formula I wherein X and Y are different and denote hydrogen or a hydroxyl group and $R^1$, $R^2$ and $R^3$ are as defined for formula I in claim 1,

i) optionally esterifying a compound of the formula I wherein X, Y, $R^2$ and $R^3$ are as defined for formula I in claim 1 and $R^1$ denotes hydrogen to a compound of the formula I wherein X, Y, $R^1$, $R^2$ and $R^3$ are as defined for formula I in claim 1 but $R^1$ does not denote hydrogen, and

j) optionally converting a compound of the formula I wherein $R^2$, $R^3$, X and Y are as defined for formula I in claim 1 and $R^1$ denotes hydrogen to a physiologically acceptable metal salt or amine salt.

3. A process for the manufacture of a pharmaceutical composition, which comprises bringing a compound as claimed in claim 1, optionally in admixture or conjunction with the pharmaceutically suitable carriers and/or stabilizers, into a therapeutically suitable dosage unit form.

4. A pharmaceutical composition which comprises an effective amount of a compound of the formula I claimed in claim 1.

5. Use of a compound of the formula I claimed in claim 1 as a medicament.